**Europäisches Patentamt**

**European Patent Office**

**Office européen des brevets**

(19)

(11) Veröffentlichungsnummer : **0 322 659 B1**

(12) **EUROPÄISCHE PATENTSCHRIFT**

(45) Veröffentlichungstag der Patentschrift :
04.12.91 Patentblatt 91/49

(51) Int. Cl.$^5$ : **A61K 31/725**

(21) Anmeldenummer : 88120960.5

(22) Anmeldetag : 15.12.88

(54) Verwendung von polysulfatierten Heparinen.

(30) Priorität : 24.12.87 DE 3744119

(43) Veröffentlichungstag der Anmeldung :
05.07.89 Patentblatt 89/27

(45) Bekanntmachung des Hinweises auf die
Patenterteilung :
04.12.91 Patentblatt 91/49

(84) Benannte Vertragsstaaten :
AT BE CH DE ES FR GB IT LI NL

(56) Entgegenhaltungen :
EP-A- 0 116 251
EP-A- 0 116 801
DE-A- 3 601 136
ARCH OF AIDS RESEARCH, Band 1, Nr. 1,
1987, Seiten 45-56; N. YAMAMOTO et al.:
"Effect of the sulfated polysaccharides on
HIV: A novel strategy of chemical modification
for HIV antivirals"
THE LANCET, Band 1, 19. Juni 1987, Seite
1379; R. UENO et al.: "Dextran sulphate, a
potent anti-HIV agent in vitro having synergism with zidovudine"
ANTICANCER RESEARCH, Band 7, Nr. 5B,
September-Oktober 1987, Seiten 1023-1038; E.
DE CLERCQ: "Perspectives for the chemotherapy of AIDS"

(56) Entgegenhaltungen :
ANTIVIRAL RESEARCH, Band 7, Juli 1987,
Seiten 361-367, Elsevier Science Publishers
B.V.; M. ITO et al.: "Inhibitory effect of dextran
sulfate and heparin on the replication of human immunodeficiency virus (HVI) in vitro"
CANCER RESEARCH, Band 38, Nr. 8, August
1978, Seiten 2401-2407; R.A. DICIOCCIO et al.:
"Inhibition of deoxynucleotide-polymerizing
enzyme activities of human cells and of simian
sarcoma virus by heparin"
BIOCHEMISTRY, Band 15, Nr. 18, 1976, Seiten
3932-3942; J.E. SHIVELY et al.: "Formation of
anhydrosugars in the chemical depolymerization of heparin"

(73) Patentinhaber : BASF Aktiengesellschaft
Carl-Bosch-Strasse 38
W-6700 Ludwigshafen (DE)

(72) Erfinder : Herr, Dieter, Dr.
Hardenburgstrasse 19
W-6701 Altrip (DE)
Erfinder : Doerper, Thomas, Dr.
Luitpoldstrasse 3
W-6719 Bissersheim (DE)
Erfinder : Daum, Lothar, Dr.
Reiherstrasse 25
W-6701 Otterstadt (DE)
Erfinder : Geiss, Karl-Heinz, Dr.
Eckbachring 46
W-6711 Heuchelheim (DE)
Erfinder : Moeller, Achim, Dr.
Wilhelm-Busch-Strasse 51
W-6703 Limburgerhof (DE)

## Beschreibung

Eine Vielzahl bösartiger lymphatisch-leukämischer Neoplasien bei Mäusen, Rindern, Vögeln, Affen und Menschen werden durch Retroviren hervorgerufen. Insbesondere wird die erworbene Immunschwäche (AIDS) beim Menschen durch ein Retrovirus, das HIV I (human immunodeficiency virus), verursacht.

Ansätze zur prophylaktischen und therapeutischen Chemobehandlung der genannten Virusinfektionen beim Menschen und anderen Säugern sind die Hemmung der Virusvermehrung durch beispielsweise Hemmstoffe der Reversen Transkriptase und/oder durch Hemmstoffe der Bindung der Retroviren an die Wirtszelle.

Handelsübliches Heparin ist ein selektiver Inhibitor der HIV-Replikation in vitro (Antiviral Res. 7 [1987] 361), es ist jedoch zur antiviralen Therapie ungeeignet, da es die Blutgerinnung hemmende Eigenschaften aufweist und Blutungen induzieren kann.

Weiter wurde gezeigt (DE-OS 3601136), daß handelsübliches Heparin die Reverse Transkriptase des Schmidt-Ruppin D (SR-D)-Virus, eines Vogelretrovirus, hemmt. Diese Hemmwirkung des Heparins konnte jedoch für die Reverse Transkriptase des HIV I nicht bestätigt werden (Antiviral Research, 7 (1987) 361 ; Conference on structure and activities of Heparin and related polysaccharides in New York, Abstract 19A, 1987, sowie eigene Versuche, siehe Tabelle 1, Seite 5).

Gegenstand der Erfindung ist die Verwendung von polysulfatierten Heparinen zur Herstellung von Arzneimitteln zur Prophylaxe und Therapie von durch Retroviren verursachten Krankheiten.

Polysulfatiertes Heparin ist Heparin, welches an beliebigen freien Hydroxy- und Aminogruppen zusätzlich sulfatiert ist, so daß der Schwefelgehalt bei mindestens 12,5% liegt. Das erfindungsgemäß verwendetete polysulfatierte Heparin besitzt vorzugsweise einen Schwefelgehalt von 13 bis 15%, insbesondere 13,5 bis 15%. Polysulfatierte Heparine mit einem Schwefelgehalt von über 16% sind praktisch nicht herstellbar.

Das polysulfatierte Heparin kann als solches oder in Form seiner Salze mit physiologisch verträglichen Basen verwendet werden. Als Salze sind insbesondere die Na-, Ca- und Mg-Salze zu verstehen. Auch Salze mit organischen Basen wie Diethylamin, Triethylamin oder Triethanolamin kommen in Betracht. Der Ausdruck "polysulfatiertes Heparin" — wie er hier verwendet wird — schließt die Salze mit ein.

Das Heparin, welches polysulfatiert wird, kann natürlich vorkommendes Heparin sein, welches ein Molekulargewicht im Bereich von 10000 bis 40000 Dalton besitzt. Durch Behandlung des natürlich vorkommenden Heparins mit Nitrit (Biochemistry 15 [1976] 3932), Enzymen (Biochem. J. 108 [1968], 647) oder mit einer Mischung aus Schwefelsäure und Chlorsulfonsäure (FR-PS 2538404) erhält man Heparine mit geringeren Molekulargewichten. Polysulfatierte Heparine mit einem Molekulargewicht von unter 1000 eignen sich nur noch schlecht zur Bekämpfung von Retroviren. Am besten geeignet sind polysulfatierte Heparine mit einem Molekulargewicht von etwa 2000 bis 20000. Die polysulfatierten Heparine mit einem Molekulargewicht von 2000 bis 9000 haben darüber hinaus den Vorteil, daß sie oral appliziert werden können.

Zur Herstellung der polysulfatierten Heparine wurden als Ausgangssubstanzen unfraktioniertes Heparin (UFH) sowie diverse, durch Nitrit-Spaltung erhaltene, niedermolekulare Heparin-Fragmente verwendet (Biochemistry 15 [1976] 3932).

Die Polysulfatierung der diversen Heparine wird vorzugsweise mit Chlorsulfonsäure in Pyridin als Lösemittel durchgeführt. Hierzu müssen die als Alkali oder Erdalkalisalze vorliegenden Heparine wie nachfolgend beschrieben in ihr entsprechendes Pyridinium-Salz überführt werden.

### Herstellung von Pyridinium-Heparinat

10 g unfraktioniertes Heparin oder 10 g depolymerisiertes niedermolekulares Heparin werden in 100 ml H2O gelöst und über eine Säule mit 50 ml Kationenaustauscher (z.B. DOWEX® MSC-1) gegeben und mit H2O eluiert. Im Eluat erscheint die entsprechende freie Heparinsäure. Diese wird sodann mit ca. 30 ml Pyridin auf pH 7 neutralisiert und anschließend lyophilisiert. Die Ausbeute beträgt ca. 11 g Heparin-Pyridinium-Salz.

Die nach obiger Methode hergestellten Pyridinium-Salze diverser Heparine stellen das zur Polysulfatierung eingesetzte Ausgangsmaterial dar. Die Intensität der Sulfatierung ist hierbei abhängig von der Menge der eingesetzten Chlorsulfonsäure. Nachfolgende Beispiele sollen die Herstellung diverser polysulfatierter Heparine näher erläutern.

### Polysulfatierung von UFH

10 g Heparin-Pyridinium-Salz wurden in 50 ml Pyridin unter Einleitung von Stickstoff eingerührt und auf 50°C erwärmt. Separat wurden 20 ml Chlorsulfonsäure (2 ml/g Heparin-Pyridinium-Salz) unter Rühren in 300 ml Pyridin eingetropft. Die Temperatur dieses Reaktionsansatzes betrug 90°C. Nach kurzem Rühren wurde die Pyridin-Chlorsulfonsäuremischung in die vorgelegte Heparin-Suspension unter heftigem Rühren einge-

2

bracht. Danach wurden weitere 60 min bei 50°C nachgerührt und auf Raumtemperatur abgekühlt.

Der körnige Niederschlag wurde abfiltriert, mit Methanol nachgewaschen, in 100 ml 0,5% NaCl-Lösung aufgenommen und mit 600 ml Ethanol ausgefällt. Der erhaltene Niederschlag wurde in $H_2O$ gelöst und das Pyridin-Heparin über 80 ml Kationenaustauscher DOWEX® MSC-1 wiederum in die freie Heparinsäure überführt. Diese wurde sodann mit 10 N NaOH auf pH 7 eingestellt und mit dem 6-fachen Volumen an Ethanol ausgefällt. Das Sediment wurde in 100 ml $H_2O$ aufgenommen, filtriert und gefriergetrocknet. Die Ausbeute betrug 10,8 g, das erhaltene Produkt (A) hatte ein Molekulargewicht ($\overline{MW}$) von 14200 Dalton und einen Schwefelgehalt von 14,8%.

Analog wurden Produkte mit folgenden Eigenschaften hergestellt :

B. ($\overline{MW}$) = 8.200, Schwefelgehalt 14,9% (aus Heparin und dem Molekulargewicht von 7.700 Dalton)
C. ($\overline{MW}$) = 5.600, Schwefelgehalt 14,1% (aus Heparin und dem Molekulargewicht von 5.100 Dalton)
D. ($\overline{MW}$) = 4.300, Schwefelgehalt 13,6% (aus Heparin und dem Molekulargewicht von 3.800 Dalton)
E. ($\overline{MW}$) = 2.800, Schwefelgehalt 14,7% (aus Heparin und dem Molekulargewicht von 2.500 Dalton)

Die Produkte A-E besitzen eine im Vergleich zu Heparin oder niedermolekularem Heparin vernachlässigbare blutgerinnungshemmende Wirkung.

Die polysulfatierten Heparine zeigen gegenüber natürlichen Heparinen oder daraus gewonnenen niedermolekularen Heparinen ein weitaus höheres Hemmpotential gegenüber der Reversen Transkriptase.

Das läßt sich mit Hilfe folgender Tests zeigen :

I. Enzymtest mit Reverser Transkriptase aus HIV 1

Im nachfolgend beschriebenen Modellsystem erfolgt die Bestimmung der Aktivität der Reversen Transkriptase in Gegenwart eines Hemmstoffes im wesentlichen nach Hansen et al. (J. Biol. Chem. 262, [1987] 12393).

Der Enzymtest hat ein Gesamtvolumen von 50 µl und enthält folgende Bestandteile : 50 mM Tris-HCl pH 7,8 ; 80 mM KCl, 8 mM $MgCl_2$, 1 mM DTT. Als Matrize enthält dieses System das synthetische Polymer poly(rA)/oligo (dT)$_{15}$ (Boehringer Mannheim) in einer Konzentration von 1 mg/ml. Diese Matrize besteht aus einer synthetischen RNA mit einer Länge von 100 bis 200 Nukleotiden, an die kurze DNA-Primer mit einer Länge von 15 Nukleotiden gebunden sind. Die DNA-Primer dienen der Reversen Transkriptase als Startstellen. Als Substrat wird in diesem Test Tritium-markiertes Thymidintriphosphat ($^3$H-dTTP, spezifische Aktivität 47 Ci-/mMol) in einer Konzentration von 100 µCi/ml verwendet. Die enzymatische Reaktion wird durch Zugabe von Reverser Transkriptase gestartet und 120 min bei 37°C durchgeführt. Die Reaktion wird durch Zugabe von 5 µl 0,5 M EDTA-Lösung beendet.

Anschließend wird die synthetisierte DNA durch Bindung an einen Anionenaustauscher von nicht-eingebauten $^3$H-dTTP-Molekülen abgetrennt. Dazu wird der gesamte Testansatz auf ein 3 × 3 cm großes DEAE-Papier aufpipettiert und getrocknet. Die an das DEAE-Papier gebundene DNA wird nun mehrmals in 0,5 M $Na_2HPO_4$ (insgesamt etwa 2 l) gewaschen, anschließend noch zweimal in destilliertem Wasser und einmal in Ethanol gewaschen.

Die an den Austauscher gebundene DNA wird unter einer Infrarotlampe getrocknet und anschließend durch Flüssigkeitsszintillationsmessung bestimmung. Als unspezifischer Hintergrund wird diejenige Radioaktivität bezeichnet, die in Abwesenheit von Reverser Transkriptase in dem oben beschriebenen Verfahren an das DEAE-Filter bindet. Der unspezifische Hintergrund beträgt ca. 100 cpm (etwa 1 o/oo) und wird gegenüber dem Meßergebnis vernachlässigt. Die zu testenden Substanzen werden in 0,9% NaCl-Lösung gelöst (1 mg/ml) und mit destilliertem Wasser so verdünnt, daß eine Endkonzentration von 50 µg/ml bzw. 5 µg/ml im Test erreicht wird.

Die Aktivität der Reversen Transkriptase in Gegenwart eines potentiellen Hemmstoffes wird angegeben in Prozent von der Aktivität ohne Hemmstoff ; bzw. man definiert die Hemmwirkung einer Substanz (%) als 100% minus Aktivität %. In Tab. 1 sind die Hemmwirkungen der einzelnen Verbindungen bei verschiedenen Konzentrationen aufgeführt.

Tabelle 1

| Substanz | (MW) | % S | Hemmung % 50 µg/ml |
|---|---|---|---|
| unfraktioniertes Heparin | 13 500 | 12 | 0 |
| niedermolekulares Heparin | 7 700 | 12 | 0 |
| niedermolekulares Heparin | 3 800 | 12 | 0 |
| A | 14 100 | 14,8 | 98 |
| B | 8 600 | 14,9 | 97 |
| C | 5 600 | 14,1 | 100 |
| D | 4 300 | 13,6 | 90 |
| E | 2 800 | 14,7 | 93 |

## II. Zytotoxizitätstest

Die toxische Bewertung der neuen Substanzen basiert auf deren zytotoxischen Effekte auf L929 Zellen. Der Zytotoxizitätstest wurde wie folgt durchgeführt:

1. 100 µl Kulturmedium mit $5 \times 10^3$ frisch trypsinierten, sich im exponnentiellen Wachstum befindenden L929-Zellen (ATCC Nr. CCl 1) wurden in die Vertiefungen einer 96-Loch-Flachboden-Kulturplatte pipettiert. Die Platte wurde über Nacht bei 37°C im Brutschrank inkubiert. Die Luft im Brutschrank enthielt 5 Vol.% $CO_2$.

Das Kulturmedium enthielt 500 ml MEM (= minimum essential medium) mit Earle's Salzen 50 ml für 30 min bei 56°C hitzeinaktiviertes foetales Kälberserum (FCS), 5 ml L-Glutamin (200 mM), 2,5 ml 100 × Non-Essential Aminosäuren, 3 ml 1M Hepes-Puffer pH 7,2 und 500 µl Gentamycin (50 mg/ml),

2. Am nächsten Tag wurden 100 µl der substanzhaltigen Probelösung in einer Endkonzentration von 500 µg Substanz/ml Kulturmedium zu den konfluenten Zellkulturen zugegeben und seriell 2-fach titiert. Auf der Kulturplatte wurden zudem eine Zellkontrolle (= nicht mit Substanzlösung behandelte Zellen) mit angelegt. Die Kulturplatte wurde dann für 48 h bei 37°C in einer Atmosphäre aus Luft mit 5 Vol.% $CO_2$ inkubiert.

3. Der Prozentsatz überlebender Zellen in den mit Substratlösung behandelten Kulturen wurde mittels der Kristallviolettfärbung bestimmt. Dazu werden die Flüssigkeiten aus den Vertiefungen durch Abschlagen der Testplatte entfernt. In jede Vertiefung wurden 50 µl Kristallviolettlösungen pipettiert. Die Kristallviolettlösung hatte folgende Zusammensetzung:

| | |
|---|---|
| 3,75 g | Kristallviolett |
| 1,75 g | NaCl |
| 161,5 ml | Ethanol abs. |
| 43,2 ml | 37% Formaldehyd |
| ad 500 ml | Wasser, welches durch Filtration über Illipore-Filter® und durch Ionenaustausch gereinigt war. |

Die Kristallviolettlösung verblieb für 20 min in den Vertiefungen und wurde dann ebenfalls abgeschlagen. Anschließend wurden die Platten jeweils 5 mal mit je 300 µl Wasser pro Vertiefungen gewaschen, um den nicht zellgebundenen Farbstoff zu entfernen. Der zellgebundene Farbstoff wurde durch Zugabe von 100 µl Reagenzlösung (50% Ethanol, 0,1% Eisessig, 49,9% Wasser) in jede Vertiefung aus den Zellen extrahiert.

4. Durch Schütteln der Platten für 5 min erhielt man in jeder Vertiefung eine gleichmäßig gefärbte Lösung. Zur Bestimmung der überlebenden Zellen wurde die Extinktion der Färbelösung in den einzelnen Vertiefungen bei 540 nm gemessen.

In diesem Test zeigen die neuen Substanzen bis zu einer Konzentration von 500 µg/ml keine Zytotoxizität auf die L929-Zellen.

Die polysulfatierten Heparinoide können in üblicher Weise oral oder parenteral (subkutan, intravenös, intramuskulär, intraperitoneal) oder als Spray verabfolgt werden.

Die Dosierung hängt vom Alter, Zustand und Gewicht des Patienten sowie von der Applikationsart ab. In der Regel beträgt die tägliche Wirkstoffdosis zwischen etwa 10 und 1000 mg/kg Körpergewicht bei oraler Gabe und zwischen etwa 0,1 und 10 mg/kg Körpergewicht bei parentraler Gabe.

Die neuen Verbindungen können in den gebräuchlichen galenischen Applikationsformen fest oder flüssig angewendet werden, z.B. alsüchlichen galenischen Tabletten, Filmtabletten, Kapseln, Pulver, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al : Pharmazeutische Technologie, Thieme-Verlag, Stuttgart, 1978).

## Beispiel 1

50 g Produkt A (Schwefelgehalt 14,8% $(\overline{MW})$ 14.200) werden in 5000 ml Wasser gelöst. Die Lösung wird mit 0,1 N Natronlauge auf pH 6,5 eingestellt. Durch Zugabe von Natriumchlorid wird die Lösung blutisotonisch gemacht. Jeweils 5 ml dieser Lösung werden in Ampullen gefüllt und sterilisiert.

Entsprechend werden Ampullen mit den Produkten B (Schwefelgehalt 14,9%, $(\overline{MW})$ 8.200), C (14,1%, 5.600), D (13,6%, 4.300) und E (14,7%, 2.800) hergestellt.

## Beispiel 2

Es werden in üblicher Weise Tabletten folgender Zusammensetzung hergestellt :

| | |
|---|---|
| 250 mg | Produkt A |
| 120 mg | Lactose |
| 60 mg | Cellulose |
| 3 mg | Magnesiumstearat |
| 52 mg | Maisstärke |
| 15 mg | Polyvinylpyrrolidon |

**Patentansprüche**

**Patentansprüche für folgenden Vertraggstaaten : AT, BE, CH, DE, FR, GB, IT, LI, NL**

1. Verwendung von polysulfatierten Heparinen zur Herstellung von Arzneimitteln zur Prophylaxe und Therapie von durch Retroviren verursachten Krankheiten.

**Patentanspruch für folgende Vertragsstaat : ES**

1. Verfahren zur Herstellung von Arzneimitteln zur Prophylaxe und Therapie von durch Retroviren verursachten Krankheiten, dadurch gekennzeichnet, daß man polysulfatiertes Heparin in eine galenische Applikationsform einarbeitet.

**Claims**

**Claim for the following Contracting States : AT, BE, CH, DE, FR, GB, IT, LI, NL**

1. Use of a polysulfated heparin for the preparation of a drug for the prophylaxis and therapy of diseases caused by retroviruses.

**Claim for the following Contracting State : ES**

1. A process for the preparation of a drug for the prophylaxis and therapy of diseases caused by retroviruses, wherein polysulfated heparin is incorporated into a pharmaceutical form of administration.

**Revendications**

**Revendication pour les Etats contractants suivants : AT, BE, CH, DE, FR, GB, IT, LI, NL**

1. Utilisation d'héparines polysulfatées pour la préparation de médicaments destinés à la prophylaxie et la thérapie de maladies provoquées par des rétrovirus.

**Revendication pour l'Etat contractant suivant : ES**

1. Procédé de préparation de médicaments destinés à la prophylaxie et la thérapie de maladies provoquées par des rétrovirus, caractérisé par le fait que l'on incorpore une héparine polysulfatée dans une forme d'application galénique.